# EUROPEAN PATENT APPLICATION

(11) **EP 1 258 488 A1**
(43) Date of publication of application: **20.11.2002**
(21) Application number: 01272252.6
(22) Date of filing: 14.12.2001
(51) Int. Cl.: C07H 1/06, C07H 15/04, C08B 37/08

(54) **PROCESS FOR PRODUCING METAL COMPLEX OF AMINOOLIGOSACCHARIDE DERIVATIVE**

(30) Priority: 26.12.2000 JP 2000039458
(71) Applicant: Nihon Medi-Physics Co., Ltd., Nishinomiya-shi, Hyogo 662-0918 (JP)
(72) Inventor: HASHIGUCHI, Yuji, c/o NIHON MEDI-PHYSICS CO., LTD., Sodegaura-shi, Chiba 299-0266 (JP); SUZUKI, Keisuke, c/o NIHON MEDI-PHYSICS CO., LTD., Sodegaura-shi, Chiba 299-0266 (JP); WADA, Masatoshi, c/o NIHON MEDI-PHYSICS CO., LTD., Sodegaura-shi, Chiba 299-0266 (JP)
(74) Representative: Wilhelms, Rolf E., Dr.
(86) International application number: JP0110983
(87) International publication number: WO02051854

(57) **Abstract**

A highly purified metal complex with an amino-oligosaccharide derivative is produced by effectively removing impurities, by-products, excess salts and the like that have been produced during the reaction steps for synthesis of the amino-oligosaccharide derivative and the steps for formation of its metal complexes. A process for producing a metal complex with an amino-oligosaccharide derivative is provided, in which a crude reaction liquid containing an amino-oligosaccharide derivative is subjected to a complexation process with a metal ion and a purification process, and the purification process comprises at least one step by a solvent extraction process, an anion exchange process, a membrane filtration process, an electrodialysis process or an adsorption process.

## Description

### Technical Field

The present invention relates to a process for producing a highly purified bulk pharmaceutical by removing impurities, unreacted compounds, by-products, excess solvents and other reagents that have been involved in reactions for producing the pharmaceutical, by means of at least one step selected from various purification processes. Specifically, the present invention relates to a process for producing a highly purified metal complex with an amino-oligosaccharide derivative, which is useful as a contrast medium for MRI (magnetic resonance imaging) or X-ray imaging.

### Background Art

In general, when a reaction is effected to synthesize a bulk pharmaceutical, a reaction product is obtained as a mixture with unreacted compounds, impurities, by-products as well as excess reagents such as solvents used in purification processes. As the purification processes for removing such excess impurities and others, have been used chromatographic processes such as high performance liquid chromatography and ion-exchange chromatography, processes using membranes such as ultrafiltration and electrodialysis, and the like. For example, Japanese Patent Laid-Open (Kokai) Hei. 11-29593 describes a method for linking an amino group-containing molecule with a chelating agent, in which a metal ion is reacted to produce the complex, after impurities, by-products and the like have been removed by the process using a membrane and/or the chromatographic process. However, the method cannot be applied in the same way to different cases, because impurities and by-products resulting from reactions for synthesis of bulk pharmaceuticals vary depending on types and syntheses of the pharmaceuticals, and the publication does not refer to any specific process although there are a variety of the membrane-using processes and the chromatographic processes.

Under the circumstances, the present invention aims to provide a process for producing a metal complex with an amino-oligosaccharide derivative, which can effectively remove impurities, by-products, excess eluents and the like that result from reaction steps for synthesis of the amino-oligosaccharide derivative and from steps for forming its metal complex, to produce a highly purified metal complex with the amino-oligosaccharide derivative.

### Disclosure of the Invention

The present invention provides a process for producing a metal complex with an amino-oligosaccharide derivative, which comprises subjecting a crude reaction liquid containing an amino-oligosaccharide derivative as represented by the following formula (1) or (2): 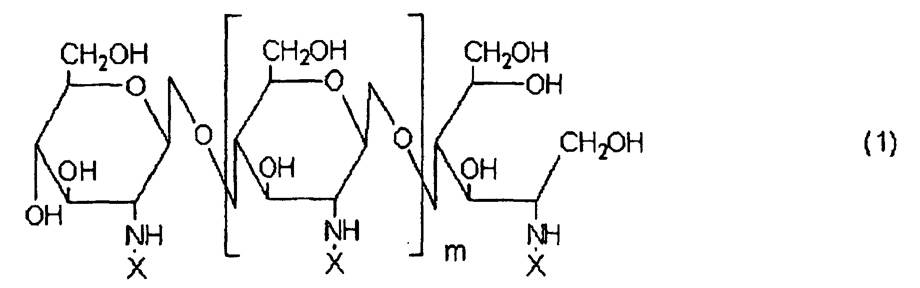 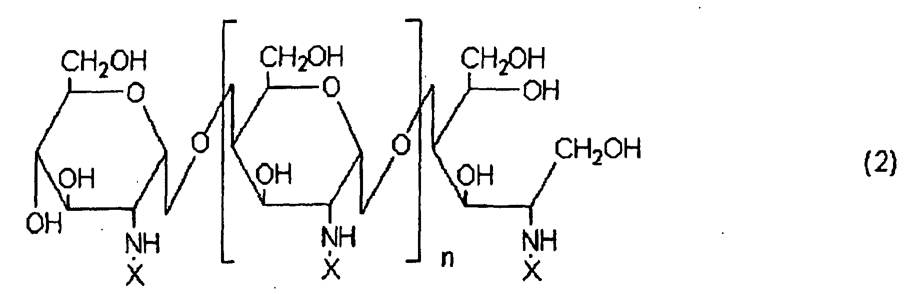 where m and n each represent an integer of 1 to 8, and X is a bifunctional ligand, to a complexation process with a metal ion and a purification process, said purification process comprising at least one step by a solvent extraction process, an anion exchange process, a membrane filtration process, an electrodialysis process or an adsorption process.

Specifically, in accordance with the process of the present invention, a highly purified metal complex with an amino-oligosaccharide derivative can be obtained by subjecting a crude reaction liquid containing an amino-oligosaccharide derivative to a complexation reaction with a metal ion and to at least one purification step by a solvent extraction process, an anion exchange process, a membrane filtration process, an electrodialysis process or an adsorption process. Preferably, said purification process comprises at least one step by a process selected from the group consisting of a methanol extraction process as the solvent extraction process; a high performance liquid chromatographic process and an open column chromatographic process as the anion exchange process; a reverse osmosis membrane process and an ultrafiltration process as the membrane filtration process; an activated carbon adsorption process as the adsorption process; and the electrodialysis process.

The purification process preferably comprises a plurality of steps. In such cases, these steps are preferably by different processes selected from the above mentioned various purification processes. Especially, combinations of a step by an anion exchange process with a step by another purification process are particularly preferable. Such combinations of steps include, for example, a combination of the step by an anion exchange process with a step by an adsorption process; a combination of the step by an anion exchange process with at least one step by a process selected from a solvent extraction process, a membrane filtration process, an electrodialysis process and an adsorption process; and a combination of the step by an anion exchange process with a step by an adsorption process and at least one step by a process selected from a solvent extraction process, a membrane filtration process and an electrodialysis process. Among the plurality of purification steps, at least one of the steps may be carried out prior to the complexation process, with the other purification steps performed subsequent to the complexation process. As a purification step which may be carried out prior to the complexation step, mention may be made of a step by a solvent extraction process which may be followed by a step by an anion exchange process which may be further followed by a membrane filtration process.

The amino-oligosaccharide derivative as represented by the above formula (1) or (2) is produced by stabilizing an amino-oligosaccharide through reductive cleavage of its reducing end, and then bonding its amino group through an amide linkage to a polyaminopolycarboxylic acid as a bifunctional ligand including ethylenediaminetetraacetic acid (hereinafter abbreviated as EDTA), diethylenetriaminepentaacetic acid (hereinafter abbreviated as DTPA), and 1,4,7,10-tetraazacyclododecane-1,4,7,10-tetraacetic acid (hereinafter abbreviated as DOTA).

The amino-oligosaccharides as mentioned above include chitosan oligosaccharides having 3 to 10 repeating units of constitutional monosaccharide and galactosamine oligosaccharides having 3 to 10 repeating units of constitutional monosaccharide. Concrete examples thereof are chitosan oligosaccharides such as chitosan trimer, chitosan tetramer, chitosan pentamer, and chitosan hexamer, and galactosamine oligosaccharides such as galactosamine trimer, galactosamine tetramer, galactosamine pentamer and galactosamine hexamer.

The crude reaction liquid in the present invention refers to a reaction liquid that contains solvents, unreacted compounds, impurities, by-products or the like resulting from the synthesis of an amino-oligosaccharide derivative, and furthermore, it may also contain metal complexes with the amino-oligosaccharide derivative, unreacted free-metals or the like resulting from the complexation process. For example, as mentioned above, an amino-oligosaccharide which has been stabilized through reductive cleavage of its reducing end (hereinafter referred to as "reducing amino-oligosaccharide") is reacted with anhydrous DTPA in an aqueous solution to form an amide linkage with DTPA. In this instance, the resulting reaction liquid contains not only the target compound in which all the amino groups of the reducing amino-oligosaccharide are bonded to DTPA, but also by-products having a molecular weight lower or higher than the target compound. Such by-products include high-molecular weight compounds formed by amide linkages or ester linkages between a plurality of reducing amino-oligosaccharides via DTPA bonded thereto; DTPA-amino-oligosaccharide compounds in which part of the amino groups of the amino-oligosaccharide are bonded to DTPA and the rest of the amino groups remain unreacted (hereinafter referred to as "defective compounds"); reducing amino-oligosaccharides cyclized by intramolecular formation of two amide linkages via an anhydrous DTPA; and DTPAs originating from unreacted anhydrous DTPA. Such a reaction liquid containing these by-products, unreacted compounds or the like is herein called a crude reaction liquid.

In the complexation process of the present production process, an amino-oligosaccharide derivative that has been bonded to a bifunctional ligand such as DTPA is allowed to coordinate with a metal ion such as paramagnetic metal ions of the lanthanoid group with atomic numbers of 57 to 70, preferably Gd or Dy, and non-paramagnetic metal ions including Pd (atomic number 82) and Bi (atomic number 83), to produce a metal complex with an amino-oligosaccharide derivative. Complexes of a paramagnetic metal ion are useful for MRI imaging, and complexes of a non-paramagnetic metal ion are useful for X-ray imaging.

The complexation reaction with a metal ion in the complexation process is carried out, for example, by adding a proper amount of a GdCl₃ solution dropwise to a liquid containing an amino-oligosaccharide derivative and stirring it while maintaining it in a pH range of 5 to 7 at room temperature. Preferably, the complexation process is carried out after the crude reaction liquid is subjected to a purification process by solvent extraction using an organic solvent to remove most of unreacted ligands like DTPA.

The purification process can be carried out in accordance with a process selected from those by a solvent extraction process, an anion exchange process, a membrane filtration process, an electrodialysis process, or an adsorption process. The anion exchange process can be carried out using an anion exchange resin, and is suitable to purify the crude reaction liquid of an amino-oligosaccharide derivative by removing therefrom by-products such as the above-mentioned lower or higher molecular weight compounds. The anion exchange resin may be used under pressure such as in a high performance liquid chromatographic process or under normal pressure such as in an open column chromatographic process using an anion exchange resin charged column, or both may be carried out. Useful anion exchange resins include polyvinyl alcohol; methacrylic materials such as polyhydroxymethacrylate and polymethacrylate; styrene-based materials such as styrene-divinylbenzene copolymer and polystyrene; silica-based materials; acrylic materials; and these materials which are coated on the surface thereof with a gel material made of a hydrophilic polymer to which a functional group such as a quaternary ammonium group, a diethylaminoethyl group, a diethylamino group, and a quaternary polyethylimine group is bonded.

The solvent extraction process is suitable to separate unreacted bifunctional ligands from the target compound and the by-products contained in the crude reaction liquid for purification. As a preferred solvent system, a water-organic solvent system is used, in which target compounds, by-products and other reacted products form an oily liquid phase to precipitate while unreacted bifunctional ligands are isolated to the upper layer separate from the oily liquid phase. Useful organic solvents include alcohols such as methanol, ethanol, propanol and butanol, of which methanol is preferable.

The adsorption process is suitable to adsorb and separate low molecular weight compounds and free metals. Useful adsorbents include carbon-based adsorbents such as activated carbon, zeolite-based adsorbents, silica-based adsorbents, alumina-based adsorbents, and adsorptive resins such as styrene-vinylbenzene copolymers and acrylic polymers, of which activated carbon is preferable.

The electrodialysis process is suitable to remove relatively low molecular weight minute impurities such as salts. In particular, it is suitable for separation and removal of trace amounts of residual NaCl originating from eluents used on ion exchange resins and trace amounts of metallic salts originating from activated carbon. Electrodialysis membranes with a molecular weight cut-off of about 100 are preferred.

The membrane filtration process includes a reverse osmosis membrane process and an ultrafiltration process, from which a suitable one is selected depending on characteristics of the membranes. The reverse osmosis membrane process is effective for concentrating the amino-oligosaccharide derivative solution by removing large amounts of eluents used in the anion exchange process. The ultrafiltration process is effective particularly for removing high molecular weight materials. When the ultrafiltration process is used for removing endotoxin at the final step of the purification process, ultrafiltration membrane modules with a molecular weight cut-off of about 20,000 are preferably employed.

Furthermore, a solution of a metal complex with the an amino-oligosaccharide derivative produced by the production process of the present invention can be dried with a freeze-drying technique or a spray-drying technique to recover the metal complex with the amino-oligosaccharide derivative in a form of powder. Such powder can be dissolved aseptically with pharmaceutically acceptable pH moderators, dissolving agents or other additives to provide contrast media for MRI and X-ray imaging.

An embodiment of the present invention is, for example, a process that uses, as the crude reaction liquid, a reaction liquid of DTPA and a chitosan trimer that has been reduced at the reducing end thereof (hereinafter referred to as reducing chitosan trimer). The crude reaction liquid contains not only the reducing chitosan trimer reacted with DTPA (hereinafter abbreviated as CH3-DTPA) but also those left unreacted and the low and high molecular weight compounds or the like as mentioned above. First, the crude reaction liquid is subjected to methanol extraction. By this operation, the target CH3-DTPA, other by-products and the like form an oily liquid phase and precipitate, while most of the unreacted DTPA moves into the liquid phase formed above the oily liquid phase. Then, the oily liquid phase is isolated. The obtained oily liquid phase is subjected to a high performance liquid chromatographic process with an anion exchange resin column to isolate CH3-DTPA, followed by a reverse osmosis membrane process to remove the high performance liquid chromatographic eluents and concentrate CH3-DTPA. To the solution containing the concentrated CH3-DTPA, is added a GdCl₃ solution dropwise to form complexes of the Gd ion with CH3-DTPA. Then, the small amounts of remaining unreacted DTPA, low and high molecular weight compounds, and the like are removed by an anion exchange process using the resin with an open-column. Then, after the free Gd ions, the eluents used for the anion exchange process, and endotoxin are successively removed by an activated carbon adsorption process, an electrodialysis process and an ultrafiltration process respectively, a highly purified aqueous solution of the CH3-DTPA-Gd complex can be obtained. The highly purified CH3-DTPA-Gd complex solution is obtained in the form of an aqueous solution with a concentration of about 20%, and as described above, can be freeze-dried or spray-dried into powder of the CH3-DTPA-Gd complex that serves as a bulk pharmaceutical for contrast media.

### Examples

The present invention will be explained in further detail by way of the following examples, but it should not be construed that the present invention is limited to these examples.

### Example 1: Methanol extraction from a crude reaction liquid

A crude reaction liquid of about 61g (50mL) containing CH3-DTPA which is a compound resulting from amide linkage of a reducing chitosan trimer with DTPA was weighed accurately and subjected to pH adjustment with 12N hydrochloric acid. Then, methanol was dropwise added thereto slowly over about 15 minutes while the solution is stirred. After completion of the addition, the solution was further stirred for 15 minutes while being kept at 30 to 35 °C, that is, the temperature at the time of the completion of the methanol addition. Then, the solution was allowed to stand for 30 minutes. As a result, the solution separated into two phases: a transparent upper layer and an oily lower layer. It was confirmed by HPLC (high performance liquid chromatography) that the upper layer mainly contained unreacted DTPA while the oily lower layer mainly contained CH3-DTPA and by-products such as the above-described low and high molecular weight compounds. Each layer was analyzed to determine the CH3-DTPA recovery rate and the unreacted DTPA removal rate. Results are shown in Table 1.

**Table 1**

| | | | | | |
|---|---|---|---|---|---|
| Number of run | 1 | 2 | 3 | 4 | 5 |
| pH (adjusted) | 3 | 4 | 5 | 3 | 3 |
| Amount of methanol (relative to crude reaction liquid) | 2 fold | 2 fold | 2 fold | 2 fold | 2 fold |
| CH3-DTPA recovery (%) | 98.6 | 93.8 | 78.6 | 100 | 100 |
| DTPA removal (%) | 32.8 | 46.8 | 63.4 | 26.8 | 17.3 |

### Example 2: Separation of CH3-DTPA by high performance liquid chromatography (HPLC)

High performance liquid chromatography (HPLC) was carried out to separate the oily liquid phase obtained in Example 1 into CH3-DTPA, low molecular weight compounds, and high molecular weight compounds. The separation conditions were as follows:
Equipment: SHIMAZU LC-8A (manufactured by Shimadzu Corporation).
Resin: Poros50 HQ (manufactured by Applied Bio System Co.)
Column size: 30 mm diameter x 250 mm length
Flow velocity: 70 mL/min
Line velocity: 10 cm/min
Detection: UV 210 nm
Elution of adsorbed materials: Stepwise elution was carried out with three sodium chloride solutions with different concentrations: 50-150 mmol/L (for elution of low molecular weight compounds), 250 mmol/L (for elution of CH3-DTPA), and 500 mmol/L (for elution of high molecular weight compounds). Results are shown in Table 2.

**Table 2**

| Number of run | | 1 | 2 | 3 |
|---|---|---|---|---|
| Eluate | low molecular weight compounds (mmol/L) | 50 | 100 | 150 |
| | CH3-DTPA (mmol/L) | 250 | 250 | 250 |
| | high molecular weight compounds (mmol/L) | 500 | 500 | 500 |
| Recovery rate of CH3-DTPA (%) | | 100 | 100 | 51.2 |
| Purity | CH3-DTPA (%) | 92.8 | 96.0 | 95.3 |
| | low molecular weight compounds (%) | 5.7 | 2.6 | 2.8 |
| | high molecular weight compounds (%) | 1.5 | 1.2 | 1.9 |

### Example 3: Salt removal by reverse osmosis membrane

A 55 liter portion of the CH3-DTPA eluate isolated by HPLC in Example 2 was used as a sample. This solution contained 0.06% CH3-DTPA while the rest was the NaCl solution that had been used as an eluent for HPLC. A polyamide module of 4 inches in diameter (D: molecular weight cut-off 300) and a sulfonated polyethersulfone module of 2 inches in diameter (E: molecular weight cut-off 500) were used. After the pH of the sample was adjusted to an appropriate value depending on the characteristics of each membrane, the sample was desalted and concentrated down to about 8 liters. Then, while purified water is added to the sample in a liquid tank to keep its volume constant, desalting was continued until conductivity became constant. Results are shown in Table 3.

**Table 3**

| | | |
|---|---|---|
| Module (molecular weight cut-off) | D (300) | E (500) |
| Recovery rate (%) | 98 | 97 |
| Desalting rate (%) | 99 | 99 |
| Permeate liquid volume (m³/day·m²) | 0.9 (1.5-2.2*) | 3.8 |
| Operating pressure (kg/m²) | 6-9 | 15 |
| pH | 6** | 11 |

| | | |
|---|---|---|
| *: Permeate liquid volume assuming an operating pressure of 15 kg/m². | | |
| **: Test was conducted at pH 6 since pH resistance was as low as 2-9. | | |

### Example 4: Purification using ion exchange resin column

An appropriate amount of a GdCl₃ solution was added dropwise to the desalted CH3-DTPA solution obtained in Example 3, and the resulting solution was stirred at pH of 5-7 at room temperature to produce CH3-DTPA-Gd complex. The solution contained not only the target CH3-DTPA-Gd complex but also trace amounts of low and high molecular weight compounds that had not been able to be removed by HPLC, and Gd complexes thereof or the like. Thus, removal of these compounds was carried out using chloride-type anion exchange resin (IRA67 manufactured by Organo), as described below.

The IRA67 (C1 type) anion exchange resin was loaded in a glass column of 21mm diameter and 58mm length. A 640 mL portion of the above-obtained CH3-DTPA-Gd complex solution (sample load: 3.2g, load factor: 0.16 g/mL (sample/resin)) was passed through the column. Then, the column was washed with 1000 mL of deionized water. Then, saline solutions of 10 mmol/L, 30 mmol/L and 50 mmol/L concentrations as eluents, were successively passed through the anion exchange resin column, and all fractions were collected and analyzed. Results are shown in Table 5. A, B and C refers to the CH3-DTPA-Gd complex, low molecular weight compounds and high molecular weight compounds, respectively. Each fraction contained about 10 mg/L of A, B and C in total, and the rest of the fraction was the saline solution used as the eluent. In Table 5, the proportions of A, B and C excluding the eluent are given as purities. Fractions F4, F5 and F6 were subjected to the subsequent purification process as the purity of A was above 97%. The recovery rate of the target component A was about 85%. Results are shown in Table 4.

**Table 4**

| Fraction | Purity of each component | | | Elution conditions |
|---|---|---|---|---|
| | A | B | C | |
| F1 | 27.5 | 72.5 | 0 | Eluate during sample loading |
| F2 | 38.5 | 61.5 | 0 | Washing with deionized water |
| F3 | 90.4 | 6.2 | 3.4 | 10 mmol/L NaCl, 1000 mL elution |
| F4 | 97.5 | 1.6 | 1.0 | 30 mmol/L NaCl, 200 mL elution |
| F5 | 98.8 | 0.4 | 0.8 | 50 mmol/L NaCl, 300 mL elution |
| F6 | 97.7 | 2.0 | 0.3 | 50 mmol/L NaCl, 600 mL elution |
| F7 | 91.0 | 8.5 | 0.5 | 50 mmol/L NaCl, 600 mL elution |
| Note: A: CH3-DTPA-Gd, B: low molecular weight compounds, C: high molecular weight compounds. | | | | |

### Example 5: Removal of low molecular weight compounds, high molecular weight compounds and free Gadolinium by activated carbon adsorption

Fractions F4, F5 and F6 obtained in Example 4 still contained free Gadolinium ions that failed to form a complex with CH3-DTPA, in addition to trace amounts of low molecular weight compounds and high molecular weight compounds. Thus, they were further removed by activated carbon adsorption. As an activated carbon powder, Taiko Activated Carbon (trade name, manufactured by Futamura Chemical Industries Co., Ltd.) was employed. The activated carbon powder was added in an amount of 7.5 g/L to a 0.9% (w/v) fraction with a purity of Component A of 98% or more, and in an amount of 15g/L to a 0.9% (w/v) fraction with a purity of Component A of 97%-98%, followed by stirring at room temperature for 60 minutes, filtration for removal of activated carbon, and analysis. Results are given in Table 5, which shows that a CH3-DTPA-Gd complex solution with a purity of 99% or more can be produced.

**Table 5**

| Sample | Purity (% content of A) | Before processing | | | After processing | | |
|---|---|---|---|---|---|---|---|
| | | A | B | C | A | B | C |
| 1 | 97-98% | 97.5 | 1.6 | 1.0 | 99.1 | 0.7 | 0.3 |
| 2 | 98% or more | 98.8 | 0.4 | 0.8 | 99.2 | 0.5 | 0.3 |
| Note: A: CH3-DTPA-Gd, B: low molecular weight compounds, C: high molecular weight compounds. | | | | | | | |

### Industrial Applicability

The present invention makes it possible to produce a highly purified metal complex with an amino-oligosaccharide derivative by effectively removing impurities, by-products, excess salts and the like that have been produced during the synthesis steps of the amino-oligosaccharide derivative.

## Claims

1. A process for producing a metal complex with an amino-oligosaccharide derivative, which comprises subjecting a crude reaction liquid containing an amino-oligosaccharide derivative as represented by the following formula (1) or (2): 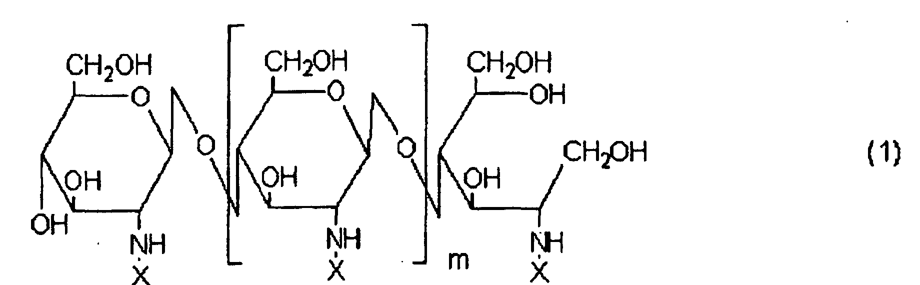 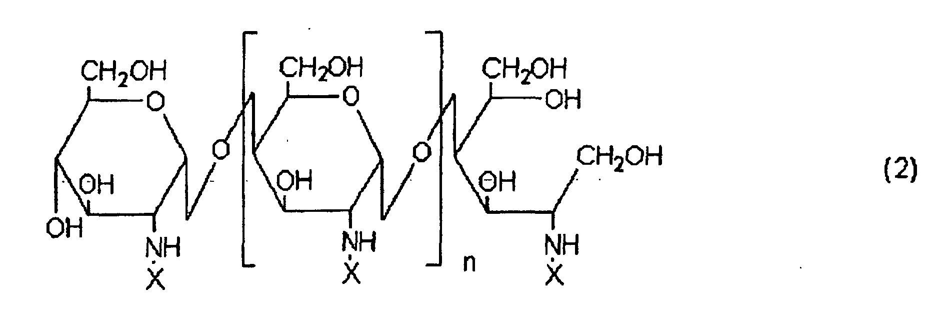 where m and n each represent an integer of 1 to 8, and X is a bifunctional ligand, to a complexation process with a metal ion and a purification process, said purification process comprising at least one step by a solvent extraction process, an anion exchange process, a membrane filtration process, an electrodialysis process or an adsorption process.

2. A process for producing a metal complex with an amino-oligosaccharide derivative according to claim 1, in which the purification process comprises a step by an anion exchange process.

3. A process for producing a metal complex with an amino-oligosaccharide derivative according to claim 1, in which the purification process comprises a step by an anion exchange process and a step by an adsorption process.

4. A process for producing a metal complex with an amino-oligosaccharide derivative according to claim 1, in which the purification process comprises at least one step by a solvent extraction process, a membrane filtration process, an electrodialysis process or an adsorption process, and a step by an anion exchange process.

5. A process for producing a metal complex with an amino-oligosaccharide derivative according to claim 1, in which the purification process comprises at least one step by a solvent extraction process, a membrane filtration process or an electrodialysis process, a step by an anion exchange process, and a step by an adsorption process.

6. A process for producing a metal complex with an amino-oligosaccharide derivative according to claim 1, in which the purification process comprises at least one step by a process selected from the group consisting of a methanol extraction process as the solvent extraction process, a high performance liquid chromatographic process and an open column chromatographic process as the anion exchange process, a reverse osmosis membrane process and an ultrafiltration process as the membrane filtration process, an activated carbon adsorption process as the adsorption process, and the electrodialysis process.

7. A process for producing a metal complex with an amino-oligosaccharide derivative according to claim 1, in which at least one step of the purification process is carried out prior to the complexation process.

8. A process for producing a metal complex with an amino-oligosaccharide derivative according to claim 7, in which the purification process that is carried out prior to the complexation process comprises a step by a solvent extraction process.

9. A process for producing a metal complex with an amino-oligosaccharide derivative according to claim 8, in which the purification process that is carried out prior to the complexation process comprises a step by a solvent extraction process and a subsequent step by an anion exchange process.

10. A process for producing a metal complex with an amino-oligosaccharide derivative according to claim 9, in which the purification process that is carried out prior to the complexation process further comprises a step by a membrane filtration process following the step by the anion exchange process.

11. A process for producing a metal complex with an amino-oligosaccharide derivative according to claim 10, in which the anion exchange process is a high performance liquid chromatographic process, and the membrane filtration process is a reverse osmosis membrane process.
